# EUROPEAN PATENT SPECIFICATION

(11) **EP 0 925 717 B1**
(45) Date of publication and mention of the grant of the patent: **15.10.2008**
(21) Application number: 98901065.7
(22) Date of filing: 30.01.1998
(51) Int. Cl.: A01M 1/20

(54) **PESTICIDE APPLICATOR**
PESTIZIDAPPLIKATOR
APPLICATEUR DE PESTICIDES

(30) Priority: 10.07.1997 JP 18528497; 25.09.1997 JP 26040497; 06.10.1997 JP 27275197; 16.12.1997 JP 34651097
(43) Date of publication of application: 30.06.1999
(73) Proprietor: Earth Chemical Co., Ltd., Tokyo 101-0053 (JP)
(72) Inventor: YAMAGUCHI, Masanaga, Ako-shi, Hyogo 678-02 (JP); KAMATANI, Mitsunobu, Ako-shi, Hyogo 678-02 (JP); MATSUSHITA, Mayumi, Ako-shi, Hyogo 678-01 (JP)
(74) Representative: Blake, John Henry Francis
(86) International application number: PCT/JP1998/000397
(87) International publication number: WO 1999/002032

(56) References cited:
- EP-A- 0 775 441
- JP-A- 7 111 850
- JP-A- 7 236 399
- JP-Y- 1 040 458
- US-A- 4 802 626

## Description

### Technical Field:

This invention relates to a technique for controlling harmful insects. More particularly, it relates to an apparatus for controlling harmful insects, especially flying harmful insects, which has a carrier having supported thereon a preparation containing a harmful insect controlling component (hereinafter referred to as a preparation-on-carrier), from which the harmful insect controlling component is vaporized making use of an air flow caused by an air blowing means; a preparation-on-carrier; and a carrier constituting the preparation-on-carrier.

### Background Art:

A great number of harmful insect controlling agents have been proposed. In practice, a proper agent is chosen from among them for the kind of harmful insects to be controlled. In particular, preparations containing a volatile active ingredient, i.e., those having a high vapor pressure at ordinary temperature have been used for flying harmful insects such as mosquitos. The problem in using volatile components is that the preparation tends to vaporize uselessly during storage or while not in use. In order to prevent a preparation from vaporizing while not used and to let a requisite amount of the preparation be released on use, harmful insect control has been carried out frequently by vaporizing a preparation under heating. For example, a mosquito coil (a spiral coil molded from a kneaded mixture of a preparation and a slow-burning base material) is lit up and burnt whereby the active ingredient is vaporized by the heat. A mat type or liquid type electric mosquito controlling apparatus comprises an appropriate base material impregnated with a preparation containing a harmful insect controlling component, in which a part of the impregnated base material is heated by means of a heater, etc. to vaporize the preparation. The insect controlling components contained in these preparations which are used under heating usually have a vapor pressure of 1 x 10⁻³ mmHg or lower at 30°C.

In this type of apparatus for vaporizing a preparation by heat, since the body of the apparatus must be made of a heat-resistant material, the employable materials are limited.

Methods for vaporizing a preparation by blowing with no heat are known. To cite an example, JP-A-U-55-954 (unexamined published Japanese utility model application) discloses an insect controlling apparatus having put therein a sublimating insect repellent, such as naphthalene, in which the volatile repellent is vaporized, and air containing the vapor is discharged through a venting hole by means of an air blower. Further, an apparatus in which a fan-shaped carrier holding an ordinary temperature volatile preparation is rotated by a motor, etc. to vaporize and diffuse the preparation is also known. In these conventional apparatus using a blowing means, because an electric motor for revolving a fan is driven by a domestic power source, the vapor is released stably by a constant voltage. However, as a matter of course the apparatus cannot be used in places where no commercial electric power is supplied. Even where commercial power is available, the place to set the apparatus is limited by the limited length of its cord. While the motor could be driven on a battery, conventional apparatus fail to run for a sufficiently long time (for example, at least about 30 days as achieved by a mat type or liquid type electric mosquito controlling apparatus) on a minimum number of easily available ordinary batteries.

Further, the conventional apparatus using a blowing means rely for adjustment of volatilization only on the on/off switch of the blower. It is therefore very likely that the active ingredient is vaporized uselessly under some conditions of use, such as the air temperature or the space in which the vapor is diffused. In such cases the active ingredient is exhausted in a shorter time than generally expected, and electric power is consumed for useless operation of the blower, which is problematical from the standpoint of energy saving whichever power source may be used, particularly where a battery is used.

The conventional apparatus using a blowing means usually has a carrier holding a preparation having a high vapor pressure, e.g., an ordinary temperature volatile preparation. In cases where a sparingly volatile component having a low vapor pressure (e.g., 1 x 10⁻³ mmHg to 1 x 10⁻⁶ mmHg at 30°C) is used, hot air should be applied for vaporization.

Furthermore, in the type of apparatus in which a preparation supported on a carrier is vaporized, duration of the preparation-on-carrier is limited due to the limited capacity of the carrier to hold the preparation.

### Disclosure of the Invention:

EP-A-0775441, cited in the European Search Report, discloses an insect control apparatus and method in which an air current is passed over an air-permeable carrier on which is supported a low-volatility insecticidal preparation. The air current is produced by a fan, which may be driven by a direct current motor. US-A-4802626, also cited in the European Search Report, discloses a honeycomb wafer which is impregnated with volatile materials, including insecticides.

An object of the present invention is to provide a harmful insect controlling apparatus and method which continues vaporizing a harmful insect controlling component at an effective rate for an extended period of time under a non-heating condition without involving useless consumption of electric power and the harmful insect controlling component.

The above objects are accomplished by:
(1) A harmful insect controlling apparatus comprising the features of claim 1,
(2) A method of controlling harmful insects which comprises the features of claim 10.

The preparation-on-carrier is a carrier having supported thereon the preparation, and the carrier suitably has a honeycomb shape, a net shape, a slit shape, a lattice shape or a shape such as perforated paper, having a plurality of vent holes which are open to the air flow in the chamber and arrayed in the plane perpendicular to the air flow, and is prepared from an organic or inorganic molding material capable of holding the preparation.

The apparatus may include a means for supplying the harmful insect controlling preparation to the carrier, and the means has a gelling material containing the preparation.

Typically, the preparation-on-carrier is a honeycomb having supported thereon said preparation, the honeycomb having a large number of through cells having a cell size of 2 to 5mm. The honeycomb may have 200 to 2500 through cells.

In the apparatus according to the present invention, the fan which can be used as a member of the blowing means includes a propeller fan, a multiblade fan (what we call a sirocco fan), and a brushless axial fan containing IC. Power sources for revolving the fan include primary batteries, solar batteries, and secondary batteries such as cadmium-nickel batteries.

In the apparatus of the invention, the preparation-on-carrier may be set in the chamber either previously or on use.

The apparatus can be provided with a means for preventing the fan from cutting one's finger while in exchanging the preparation-on-carrier. For example, a means for detecting the chamber being opened can be provided so that the blowing means may be stopped by the signals from the detecting means.

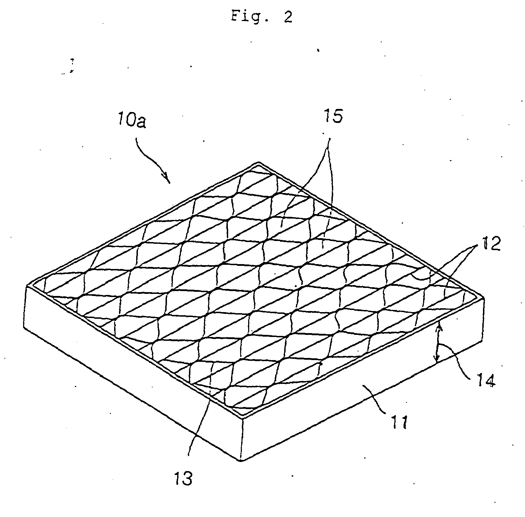

The carrier for supporting the preparation preferably has a simple structure having good ventilation. For example, the carrier includes a honeycomb shape as shown in Fig. 2, a drainboard shape, a bellows shape, a net shape, a slit shape, a lattice shape, a shape such as perforated paper, and the like.

The material fabricating the carrier is not particularly limited as long as it can hold the preparation. Materials capable of continuously releasing the preparation at a given vaporization rate over an expected period of time are preferred to those which releases the preparation in a short time. Suitable materials include paper (filter paper, pulp, paperboard, etc.), resins (polyethylene, polypropylene, polyvinyl chloride, oil-absorbing polymers, etc.), ceramics, glass fiber, carbon fiber, chemical fibers (polyester, polyamide, acrylic resins, vinylon, polyethylene, polypropylene, etc.), natural fibers (cotton, silk, wool, flax, etc.), nonwoven fabric made of glass fiber, carbon fiber, chemical fiber, natural fiber, etc., porous glass materials, and metal net. The effect of the carrier is not influenced by the shape of the individual cells. For example, the cell can have a hexagonal shape (i.e., a honey comb shape), a circular shape or an S-shape.

The preparation which can be supported on the carrier is not particularly limited as far it comprises an active ingredient, such as a harmful insect controlling component including an insecticide and an insect repellant, that vaporizes in the air to produce a given effect. Preparations from which the active ingredient is vaporized by applying an air flow are particularly preferred. Suitable examples of the harmful insect controlling components such as insecticides and insect repellents are shown below.
dl-3-Allyl-2-methyl-4-oxo-2-cyclopentenyl dl-cis/trans-chrysanthemate (general name: allethrin; trade name: Pynamin, produced by Sumitomo Chemical Co., Ltd.)
dl-3-Allyl-2-methyl-4-oxo-2-cyclopentenyl d-cis/trans-chrysanthemate (trade name: Pynamin Forte, produced by Sumitomo Chemical Co., Ltd.)
dl-3-Allyl-2-methyl-4-oxo-2-cyclopentenyl d-trans-chrysanthemate (trade name: Bioallethrin, produced by Uclaf Co., Ltd.)
d-3-Allyl-2-methyl-4-oxo-2-cyclopentenyl d-trans-chrysanthemate (trade name: Exthrin, produced by Sumitomo Chemical, and Esbiol, produced by Uclaf Co., Ltd.)
(5-Benzyl-3-furyl)methyl d-cis/trans-chrysanthemate (general name: resmethrin; trade name: Crysron Forte, produced by Sumitomo Chemical Co., Ltd.)
5-Propargyl-2-furylmethyl d-cis/trans-chrysanthemate (general name: furamethrin; trade name: Pynamin D Forte, produced by Sumitomo Chemical Co., Ltd.)
(+)-2-Methyl-4-oxo-3-(2-propenyl)-2-cyclopentenyl (+)-cis/trans-chrysanthemate (general name: prallethrin; trade name: Etoc, produced by Sumitomo Chemical Co., Ltd.)
dl-3-Allyl-2-methyl-4-oxo-2-cyclopentenyl dl-cis/trans-2,2,3,3-tetramethylcyclopropanecarboxylate (general name: terallethrin; produced by Sumitomo Chemical Co., Ltd.)
(1,3,4,5,6,7-Hexahydro-1,3-dioxo-2-isoindolyl)methyl dl-cis/trans-chrysanthemate (general name: phthalthrin; trade name: Neopynamin, produced by Sumitomo Chemical Co., Ltd.)
(1,3,4,5,6,7-Hexahydro-1,3-dioxo-2-isoindolyl)methyl d-cis/trans-chrysanthemate (trade name: Neopynamin Forte, produced by Sumitomo Chemical Co., Ltd.)
3-Phenoxybenzyl-d-cis/trans-chrysanthemate (general name: phenothrin; trade name: Sumithrin, produced by Sumitomo Chemical Co., Ltd.)
3-Phenoxybenzyl-dl-cis/trans-3-(2,2-dichlorovinyl)-2,2-dimethyl-1-cyclopropanecarboxylate (general name: permethrin; trade name: Eksmin, produced by Sumitomo Chemical Co., Ltd.)
(±)-α-Cyano-3-phenoxybenzyl (+)-cis/trans-chrysanthemate (general name: cyphenothrin; trade name: Cokilant produced by Sumitomo Chemical Co., Ltd.)
1-Ethynyl-2-methyl-2-pentenyl dl-cis/trans-3-(2,2-dimethylvinyl)-2,2-dimethyl-1-cyclopropanecarboxylate (general name: empenthrin; trade name: Vaporthrin, produced by Sumitomo Chemical Co., Ltd.)
d-Trans-2,3,5,6-tetrafluorobenzyl-3-(2,2-dichlorovinyl)-2,2-dimethyl-1-cyclopropanecarboxylate (general name: transfluthrin)
1-Ethynyl-2-methyl-2-pentenyl 3-(2,2-dichloroethenyl)-2,2-dimethylcyclopropanecarboxylate.

Compounds which are structurally similar to the compounds listed above and have practically the same effects are also useful. For empenthrin having two methyl groups at the 3-position, for instance, analogues having other alkyl groups, unsaturated alkyl groups or halogen atoms in place of the methyl groups can be used.

In addition, insecticides such as Fipronil, S-1295, and S-41311; juvenile hormones such as methoprene (isopropyl(2E-2E)-11-methoxy-3,7,11-trimethyl-2,4-trimethyldodeca-2,4-dienoate), pyriproxyphene (82-[1-methyl-2-(phenoxyphenoxy)ethoxy]pyridine); and chitin formation inhibitors such as diflubenzuron (1-(4-chlorophenyl)-3-(2,6-difluorobenzoyl)urea) and teflubenzuron (1-(3,5-difluorobenzoyl)urea) can be used.

Of these compounds, those which are sparingly volatile at ordinary temperature are preferred. In particular, empenthrin, pramethrin, resmethrin, Esbiol, furamethrin, terallethrin, transfluthrin, and S-Hydroprene are still preferred.

The harmful insect controlling components can be used either individually or as a combination of two or more thereof. Analogues of the above-enumerated compounds are also useful.

Of the compounds those which are volatile at ordinary temperature can be made sparingly volatile to produce their effect for a prolonged period of time by providing a cover for volatility control or by compounding with a volatility controlling agent comprising a hydrocarbon such as polybutene, isoparaffin or n-paraffin or an ester such as hexyl laurate, isopropyl myristate or butyl phthalate.

The preparation can contain aromatics, bactericides, and so forth in addition to or in place of the harmful insect controlling components. Any of natural perfumes and artificial perfumes or compounded perfumes can be used as aromatics with no particular limitation.

Examples of suitable natural perfumes include animal perfumes such as musk, civet, and ambergris; and vegetable perfumes such as abies oil, ajowan oil, almond oil, angelica root oil, basil oil, bergamot oil, birch oil, rosewood oil, cajuput oil, cananga oil, capsicum oil, caraway oil, cardamon oil, cassia oil, celery oil, cinnamon oil, citronella oil, cognac oil, coriander oil, cubeb oil, cumin oil, camphor oil, jill oil, estragon oil, eucalyptus oil, fennel oil, garlic oil, ginger oil, grapefruit oil, hop oil, juniper berry oil, laurel leaf oil, lemon oil, lemongrass oil, lovage oil, mace oil, nutmeg oil, mandarin oil, tangerine oil, mustard oil, Japanese mint oil, neroli oil, onion oil, pepper oil, orange oil, sage oil, star anis oil, turpentine oil, wormwood oil, and vanilla bean extract.

The artificial perfumes include synthetic ones and extracts. Examples of suitable artificial perfumes include hydrocarbons such as pinene and limonene; alcohols such as linalool, geraniol, citronellol, menthol, borneol, benzyl alcohol, anis alcohol, and β-phenylethyl alcohol; phenols such as anethole and eugenol; aldehydes such as n-butyraldehyde, isobutyraldehyde, hexyl aldehyde, heptyl aldehyde, n-nonyl aldehyde, nonadienal, citral, citronellal, benzaldehyde, cinnamaldehyde, heliotropin, and vanillin; ketones, such as methyl amyl ketone, methyl nonyl ketone, diacetyl, acetylpropionyl, acetylbutyryl, carvone, menthone, camphor, acetophenone, p-methylacetophenone, and ionone; lactones or oxides, such as amylbutyrolactone, ethyl methylphenylglycidate, γ-nonyllactone, coumarin, and cineole; and esters, such as methyl formate, isopropyl formate, linalyl formate, ethyl acetate, octyl acetate, menthyl acetate, benzyl acetate, cinnamyl acetate, butyl propionate, isoamyl acetate, isopropyl isobutyrate, geranyl isovelerate, allyl caproate, butyl heptylate, octyl caprylate, methyl heptynecarboxylate, ethyl pelargonate, methyl octynecarboxylate, isoamyl caprate, methyl laurate, ethyl myristate, ethyl benzoate, benzyl benzoate, methyl phenylacetate, butyl phenylacetate, methyl cinnamate, cinnamyl cinnamate, methyl salicylate, ethyl anisate, methyl anthranilate, ethyl pyruvate, and ethyl α-butylbutyrate.

These perfume components can be used either individually or as a blend of two or more thereof. Examples of blend perfumes are a combination of musk as a natural perfume and pinene as a synthetic perfume and a combination of civet as a natural perfume and limonene as a synthetic perfume.

The preparation-on-carrier can be prepared by applying a liquid preparation to a carrier by dripping, impregnation, spraying, printing (inclusive of jet printing), brush coating, or a like coating technique. It is also possible to adhere a preparation in a solid state to the carrier. Where the preparation is too viscous to be absorbed, it is diluted with a solvent to a concentration practical for impregnation. A non-liquid state preparation may be rubbed into the carrier or printed on the carrier. The preparation can be supported on the carrier either directly by absorption, or it can be once absorbed by a supporting assistant such as a gel substance. In the latter case, the supporting assistant is supported by the carrier. Further, a liquid preparation can be supplied to its carrier continuously through a wick (string or tape) or by dripping.

### Brief Description of Drawings:

Fig. 1 is a schematic view of a preferred harmful insect controlling apparatus according to the present invention.
Fig. 2 is a perspective view of a preferred carrier for use in the present invention.
Fig. 3 shows graphs of running time vs. vaporization rate of active ingredient in the apparatus of Fig. 1 driven by different motors.
Fig. 4 shows a graph of running time vs. vaporization rate of active ingredient in the apparatus of Fig. 1 driven on different power sources.
Fig. 5 schematically illustrates a testing room in which the performance of the apparatus of Fig. 1 is tested.
Fig. 6 shows a graph of air temperature vs. amount of active ingredient vaporized from the apparatus of Fig. 1 operated with or without control on vaporization.
Figs. 7 through 11 each furnish a perspective view and a plane view of preferred carriers for supporting a preparation for use in the present invention.
Fig. 12 schematically illustrates a silica gel trap used for measuring the amount of a vaporized active ingredient.
Fig. 13 shows a graph of honeycomb cell size vs. vaporization rate of active ingredient.
Fig. 14 is a schematic view of another preferred harmful insect controlling apparatus according to the present invention, in which a pressure fan is provided.
Fig. 15 is a graph showing the relationship between (a) the distance between the pressure fan and the honeycomb in the apparatus of Fig. 14 and (b) the air flow at the air outlet.
Fig. 16 is a graph showing the relationship between (a) the distance between the fan and the honeycomb in the apparatus of Fig. 14 in which a suction fan is provided and (b) the air flow at the air outlet.
Fig. 17 is a graph showing the relationship between (a) the distance between the pressure fan or the suction fan and the honeycomb in the apparatus of Fig. 14 in which a suction fan or a pressure fan is provided and (b) the vaporization rate of the active ingredient.
Fig. 18 is a schematic view of still another preferred harmful insect controlling apparatus according to the present invention.
Fig. 19 is a schematic view of a preferred carrier for supporting a preparation for use in the present invention.
Fig. 20 is a perspective view and a plane view indicating a "cell size" of a honeycomb structure in the conventional sense.

Preferred embodiments of the present invention will be illustrated with reference to the accompanying drawings. Unless otherwise noted, all the dimensions, materials, shapes and positional relations of the component parts hereinafter described are given only for illustrative purposes but not for limitation.

### EXAMPLE 1

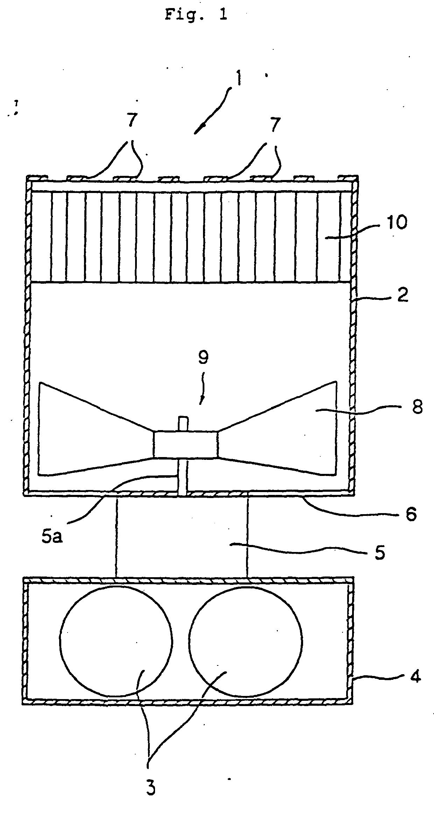

A harmful insect controlling apparatus 1 shown in Fig. 1 comprises a battery box 4 containing batteries 3, a direct current motor 5, and a chamber 2. The battery box 4 has terminals (not shown) which are in contact with the electrodes of the batteries 3 and connected to the direct current motor 5 via an operation control circuit (not shown). The battery box 4 and the chamber 2 are connected together via, for example, the casing of the direct current motor 5 as shown in Fig. 1. The motor 5 has a drive shaft 5a vertically projecting from the bottom of the chamber 2 into the inside of the chamber. The chamber 2, which is hollow, has an air inlet 6 in the bottom and an air outlet 7 in the top. A preparation-on-carrier 10, which comprises a carrier having supported thereon a preparation, is fitted in the chamber 2 right under the air outlet 7.

There is a fan 8 right above the air inlet 6, fixed to the drive shaft 5a of the motor 5. The fan 8 blows a current of air taken in through the inlet 6 across the chamber toward the outlet 7. The air flow from the outlet 7 ranges from 0.2 to 6 l/sec. While the axis of revolution of the fan 8 in Fig. 1 is fixed, the vertical position of the fan 8 may be adjustable, or the fan 8 may swivel.

The fan 8 comprises a plurality of tilt blades radially arranged on the drive shaft 5a. The fan 8 and the direct current motor 5 thus make up an axial blowing means 9. A multiblade fan (what we call a sirocco fan) having a plurality of blades around a cylindrical runner in parallel with the drive shaft can also be used as a blowing means. In this case, the fan blows an air flow in the direction perpendicular to the drive shaft 5a. In addition, a brushless axial fan containing IC can be used as well.

The weight of the fan 8 is less than 30 g in view of the battery power. More specifically, a propeller fan weighing about 3 to 17 g and a sirocco fan weighing about 7 to 15 g are suitable. Specifications of fans satisfying the weight condition are shown below.
Propeller fan:
   (1) diameter: 56 mm; 11-bladed
   (2) diameter: 48 mm; 11-bladed
   (3) diameter: 74 mm; 7-bladed
Sirocco fan:
   (1) diameter: 58 mm; 12-bladed (blade width: 23 mm; blade length: 10 mm)
   (2) diameter: 45 mm; 12-bladed (blade width: 20 mm; blade length: 8 mm)
   (3) diameter: 54 mm; 8-bladed (blade width: 13 mm; blade length: 8 mm)
Brushless axial fan:
   (1) diameter: 56 mm; 11-bladed

Where an axial blowing means is used as in Fig. 1, the air sucked in from the air inlet 6 has a characteristic that the speed of flow gets slower toward the center of the fan and faster toward the periphery of the fan. Accordingly, the amount of air applied to the preparation-on-carrier 10 is smaller in the central portion and larger in the peripheral portion. It means that the diffusion of the vapor of the preparation is non-uniform throughout the preparation-on-carrier 10. To cope with this problem, a flow regulator (crossing plates) can be provided in the air passageway between the fan 8 and the preparation-on-carrier 10 to make the air flow applied to the preparation-on-carrier 10 uniform. The air having been applied to the preparation-on-carrier 10 and now containing the vapor of the active ingredient is discharged outside through the outlet 7, whereby the active ingredient of the preparation is released from the preparation-on-carrier 10, made to flow, let out through the air outlet, and diffused in the atmosphere. The carrier can have a flow regulating function, or the flow regulator which is provided separately can have a function of holding the preparation.

In this example, while the preparation-on-carrier is placed on the exhaust side of the fan 8, it may be on the air inlet side of the fan.

The performance of the harmful insect controlling apparatus 1 was tested as follows.

### Test 1:

A paper-made honeycomb structure (66 mm x 66 mm x 15 mm (t)) the cells of which look like the section of a pile of corrugated cardboard was impregnated with a preparation containing 2 g of transfluthrin as a harmful insect controlling component to prepare a preparation-on-carrier 10. The preparation-on-carrier was set in the apparatus 1 equipped with a sirocco fan (diameter: 58 mm; blade width: 23 mm) and a direct current motor consuming a current of 25 mA with no load applied. The apparatus 1 was operated on two size-D alkali batteries connected in series. For comparison, the motor was replaced with a direct current motor whose current consumption is 160 mA with no load applied.

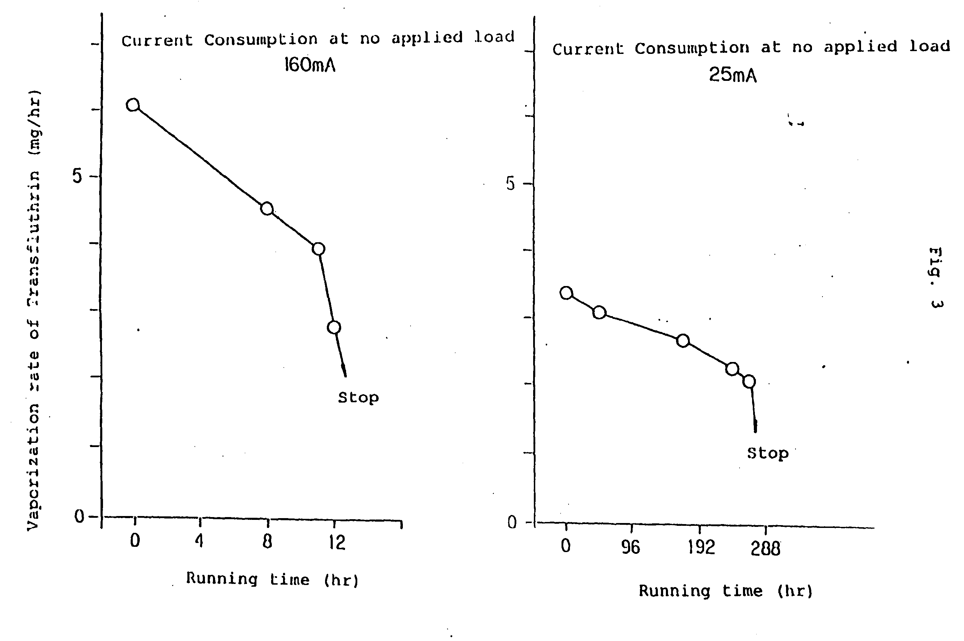

The test results are shown in Fig. 3. It is obvious that the apparatus using a direct current motor having a current consumption of 25 mA with no load applied runs longer than the apparatus using a direct current motor having a current consumption of 160 mA with no load applied while producing a sufficient amount of transfluthrin vapor.

### Test 2:

The same preparation-on-carrier as used in test 1 was set in the apparatus 1 equipped with a direct current motor having a current consumption of 6 mA with no load applied and a sirocco fan (diameter: 58 mm; blade length: 23 mm). The apparatus was run on 2 size-D manganese dry batteries connected in series or, for reference, at a constant voltage of 3 V for 12 hours a day for 30 days. The vaporization rate of transfluthrin was found to be about 1.5 mg/hr with an air flow of 2 l/sec.

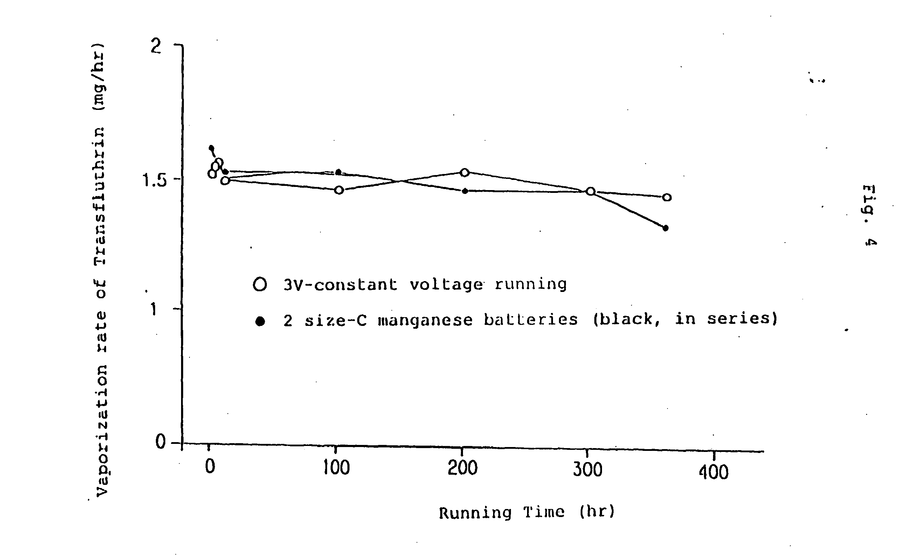

The test results are shown in Fig. 4. It can be seen that the apparatus running on batteries produces transfluthrin vapor at a stable vaporization rate for a long time equally to the apparatus run at a constant voltage of 3 V.

### Test 3:

The same honeycomb structure as used in test 1 was impregnated with a preparation containing 1 g of transfluthrin. The preparation-on-carrier was set in the same apparatus as used in test 2 equipped with 2 size-C manganese batteries connected in series. The apparatus was switched on and off at 2-minute intervals for 12 hours a day for consecutive 30 days, and the amount of vaporized transfluthrin was measured with time. As a result, transfluthrin was vaporized in an effective amount for the 30 day period similarly to the results of test 2.

### Test 4 (Effect on Culex pipiens pallens):

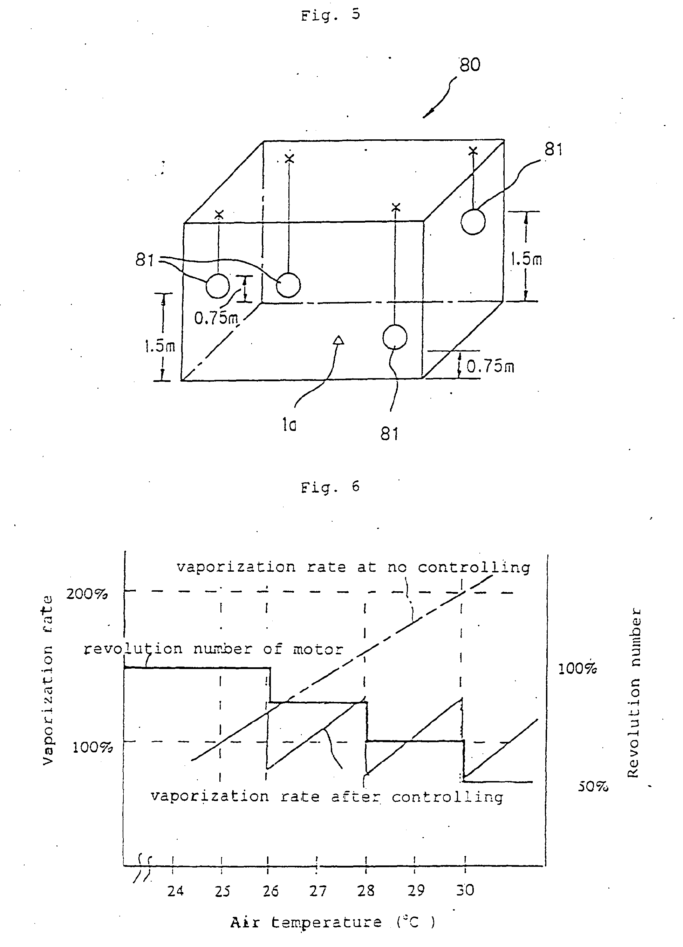

The effectiveness of the harmful insect controlling apparatus 1 was tested as follows. As shown in Fig. 5 and Table 1, a harmful insect controlling apparatus 1a (the same as the apparatus 1 of Fig. 1) was placed on the center of the floor of a test room 80 having a capacity of 22.8 m³ (2.5 m x 3.5 m x 2.6 m (height)). A 25 cm-square cage 81 made of stainless steel net in which 15 female imagoes of *Culex pipiens pallens* were released was hung from the ceiling at each corner; two cages on one diagonal were 0.75 m high from the floor, and other two on the other diagonal were 1.5 m high from the floor. The room temperature was kept at 25±1°C. The number of knocked down insects was counted with time. During the testing, air in the room was sucked up, and the trapped active ingredient (transfluthrin) was quantitatively analyzed by gas chromatography using dibutyl phthalate as an internal standard. Two hours later, the insects were transferred into a clean plastic container and fed on a 1% aqueous sugar solution in another room kept at about 25°C. After 24 hours from the start of testing, the number of dead insects was counted to obtain the death rate. Under the above conditions the air flow of the apparatus 1a was 2 l/sec, and the rate of transfluthrin vaporization was 0.4 mg/hr.

For comparison, the same test was carried out using a conventional liquid type electric mosquito controlling apparatus in place of the apparatus 1a.

The average results (n=4) are shown in Table 1 below.

**TABLE 1**

| Effect on *Culex pipiens pallens* | | | |
|---|---|---|---|
| | KT₅₀ (min) | KT₉₀ (min) | Death Rate (%) |
| Apparatus 1a | 43 | 58 | 91.8 |
| Liquid type mosquito controlling apparatus | 42 | 60 | 62.5 |

It is seen from Table 1 that the apparatus 1a achieves KT₅₀ in about 43 minutes and KT₉₀ in about 58 minutes, proving superior in knock-down effect to the comparative liquid type electric mosquito controlling apparatus and that the death rate achieved by the apparatus 1a is as high as 90% or more. Further, the apparatus 1a stably showed a knock-down effect and a killing effect at every time of measurement.

Even when the vaporization rate was reduced to 0.2 mg/hr, sufficient insecticidal effects were obtained.

Since the apparatus 1a vaporizes and diffuses the active ingredient rapidly by forced air blowing, it is expected that the active ingredient is diffused in a wide space more rapidly than by a conventional evaporating apparatus.

Because the harmful insect controlling apparatus of this example is operated by a direct current motor on easily available batteries, it enjoys improved convenience of use with no restriction in place to set. The direct current motor having a current consumption of not more than 100 mA with no load applied makes it possible to run the apparatus for an extended period of time on batteries, bringing about improved economy.

### EXAMPLE 2

The harmful insect controlling apparatus 1 shown in Fig. 1 was fitted with an infrared sensor (not shown). The blowing means 9 comprising the motor 5 and the fan 8 was controlled based on the signals from the sensor to control the conditions of the air flow against the preparation-on-carrier 10 so that the vaporization rate of the active ingredient might be optimized for the air temperature. The infrared sensor and a means for controlling the blowing means 9 based on the signals from the sensor constitute a means for regulating the air flow.

The same carrier and harmful insect controlling component as used in Example 1 can be used in Example 2. In order to facilitate application of a liquid preparation to the carrier by impregnation, an organic solvent, such as fatty acid esters (e.g., isopropyl myristate, isopropyl palmitate, and hexyl laurate), isopropyl alcohol, polyethylene glycol, and deodorized kerosine, can be used if desired as an additive for reducing the viscosity.

The amount of the harmful insect controlling component and/or other various components to be supported on the carrier is not particularly limited. A preferred minimum is such for assuring a minimum vaporization rate of 0.1 mg/hr. While the harmful insect controlling component is usually infiltrated into the carrier up to saturation, the amount to be infiltrated can be increased substantially by connecting a separate container for replenishment to the carrier.

To control the air flow conditions so as to optimize the vaporization rate of the harmful insect controlling component for the air temperature means to control the flow rate, the pressure and the like of the air flow in conformity to the air temperature. Such control can be carried out according to seasons, daytime or nighttime, etc.

The following test was conducted to examine the effects of the apparatus of Example 2.

### Test 5:

A paper-made honeycomb structure (66 mm x 66 mm x 15 mm (t)) the cells of which look like the section of a pile of corrugated cardboard was impregnated with a preparation to prepare a preparation-on-carrier containing 100 mg/g to 700 mg/g of transfluthrin as a harmful insect controlling component.

Fig. 6 is a graph showing changes in amount of vaporized transfluthrin with changes in air temperature. The thin solid line indicates the amount of transfluthrin vaporized from the apparatus of Example 2 when the number of revolution of the fan is controlled, and the broken line is the amount of transfluthrin vaporized with a constant number of revolution of the fan. As shown by the thick solid line, the number of revolution of the direct current motor decreases stepwise in inverse proportion to the increase of air temperature. That is, taking the number of revolution at temperatures below 26°C as 100%, the number of revolution drops to about 83% at 26°C, about 64% at 28°C, and about 50% at 30°C. With the drop in number of revolution of the motor, the amount of vaporized harmful insect controlling component (shown by the thin solid line) decreases by about 40% for every 1°C rise from 26°C. That is, the amount of the vaporized component is maintained within a prescribed range with slight increases and decreases in a temperature changing from 26°C to 30°C.

Where the speed of the motor is not controlled, on the other hand, the amount of vaporized transfluthrin increases linearly with an elevating air temperature as indicated by the broken line, showing an increase from about 120% at 26°C to about 200% at 30°C. It is understood from these results that the apparatus according to Example 2 minimizes useless vaporization of the active ingredient as compared with the apparatus with no control on the direct current motor.

The following modifications (1) to (7) can be added to the apparatus of Example 2. Reference is made to Fig. 1.
(1) The infrared sensor is replaced with a thermometer (not shown), a reading of which is automatically taken. The direct current motor 5 of the blowing means 9 switches between continuous running and intermittent running based on the reading.
(2) The infrared sensor is replaced with a thermometer (not shown) and a manual control displaying temperature on its dial (not shown). The control is turn manually based on a reading of the thermometer to control the number of revolution of the direct current motor 5.
(3) The infrared sensor is replaced with a thermometer (not shown) and a manual control displaying temperature on its dial (not shown), and the air inlet 6 and the air outlet 7 of the chamber 2 are each provided with a shutter (not shown) to adjust the respective open areas. The control is turn manually based on a reading of the thermometer to control the number of revolution of the direct current motor 5 and the open area each of the air inlet 6 and the air outlet 7.
(4) In addition to the infrared sensor a shutter (not shown) is provided at the air inlet 6 and the air outlet 7 in the chamber 2 to adjust the respective open areas. The shutter is controlled based on the air temperature automatically detected by the sensor.
(5) The infrared sensor is displaced with a manual control (not shown) displaying spaces on its dial, and the air inlet 6 and the air outlet 7 of the chamber 2 are each provided with a shutter (not shown) to adjust the respective open areas. The control is turn manually based on the space in which the apparatus is used to adjust the open area each of the air inlet 6 and the air outlet 7.
(6) A season selector switch (not shown) is provided in place of the space selecting control according to the modification (5). The selector switch is adjusted to the season (e.g., one of 4 seasons) when the apparatus is used thereby to control the open area each of the air inlet 6 and the air outlet 7.
(7) A daytime/nighttime selector switch (not shown) is provided in place of the space selecting control according to the modification (5). The selector switch is adjusted to "daytime" or "nighttime" on the dial thereby to control the open area each of the air inlet 6 and the air outlet 7.

The other component parts and effect of the above-described modified apparatus are the same as those of the apparatus of Example 2.

According to the modifications (1) and (2), the number of revolution of the direct current motor 5 is controlled based on the automatically or manually furnished temperature information, whereby the air flow and pressure are controlled to optimize the vaporization rate for the air temperature.

According to the modification (3), the number of revolution of the direct current motor 5 and the open areas of the air inlet 6 and the air outlet 7 are controlled based on the manually furnished temperature information, whereby the air flow and pressure are controlled to optimize the vaporization rate for the air temperature.

According to the modifications (4) to (7), the open areas of the air inlet 6 and the air outlet 7 are controlled based on the information from an infrared sensor or a space, season or daytime/nighttime selector, whereby the air flow and pressure are controlled to optimize the vaporization rate for the space, season or time of use.

Since the vaporization rate of the harmful insect controlling component is optimized according to the air temperature, the space of diffusion, and the season or time of use, useless vaporization of the component can be minimized. Besides, useless revolution of the direct current motor 5 is avoided to afford an energy saving and to extend the service life of the batteries 3. As a result, the apparatus continues vaporizing an effective amount of the harmful insect controlling component for an extended period of time. In other words, the active ingredient is vaporized in an effective amount in agreement with the environmental conditions for a long time. The disadvantage that the active ingredient is exhausted in a shorter time than a generally expected duration can be eliminated.

While the preparation-on-carrier 10 is disposed on the exhaust side of the fan 8 (pressure fan type) in the above-described embodiments, it may be placed on the inhalation side of the fan 8 (suction fan type). Useful fans include a rotary fan, a sirocco fan, a piezoelectric fan, etc. A compressor may be used in place of the fan 8.

The harmful insect controlling apparatus of Example 2 has an air flow regulating means which is designed to control the conditions of the air flow applied to the preparation-on-carrier so as to optimize the vaporization rate of the active ingredient according to the environment. Therefore, the amount of the active ingredient vaporized per unit time can be optimized according to the environment whereby effective vaporization and diffusion of the active ingredient can be secured for a prolonged period of time.

### EXAMPLE 3

A honeycomb having a cell size of 2 to 5 mm (the term "cell size" as used herein will be defined later) is used as a carrier for supporting a preparation in the harmful insect controlling apparatus 1 shown in Fig. 1. Such a honeycomb includes a paper core used mainly for sandwich panels. A paper core is a structure having a void area ratio of 90% or more, which is fabricated of paper with an adhesive to have a large number of through cells. The through cells each have a section of a hexagon, a circle or a nonequilateral polygon, sharing part of their sides. That is, a paper core which has a great number of through cells and an extremely high void area ratio is advantageous in that a large surface area is secured for supporting a preparation on and that air can be blown through the cells with little resistance. The structure of the honeycomb is not particularly limited as long as it can hold the preparation. A honeycomb having a larger thickness has a larger surface area and a higher resistance to ventilation. From this viewpoint, a suitable thickness of a honeycomb whose cells have a shape like, for example, the section of a pile of corrugated cardboard is about 2 to 15 mm. The thickness range is subject to variation according to the material of the honeycomb.

Fig. 2 shows a carrier 10a comprising a honeycomb structure framed in a square frame 11. The honeycomb has a large number of cells through the thickness direction 14, looking like the section of a pile of corrugated cardboard. More specifically, the honeycomb is made up of a plurality of tape liners 12 which are in planar parallel with each other at regular intervals and a plurality of corrugated tapes 13 each sandwiched in between each adjacent two tape liners 12 with an adhesive to form through cells 15. It is preferable for securing strength that the peaks of a corrugated tape face the valleys of an adjacent corrugated tape with a liner tape therebetween. The cell size is 2 to 5 mm. The size of the frame 11 is subject to variation depending on a desired amount of the preparation to be supported or to be vaporized per unit time. A preferred number of cells of a honeycomb carrier ranges 200 to 2500.

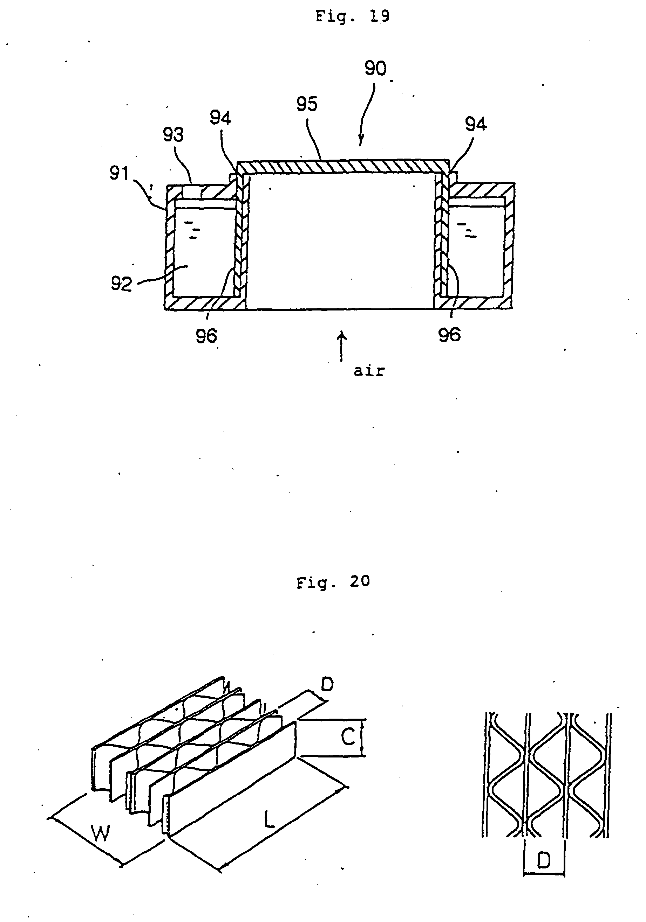

In general, the size of a cell of a honeycomb structure is represented by the distance between centers, in thickness direction, of two partitioning walls forming cells as specified in JIS A 6931 (1978). Fig. 20, which is a quotation from JIS A6931, shows a perspective view and a partial plane view of a honeycomb structure, in which the cell size is represented by symbol D. The cell size has been so defined because of practical applicability even to non-circular cells. However, with the cell size being equal, cells formed of thicker paper (partitions) have a smaller inner diameter. Considering that the size of the cells directly influences vaporization of the preparation supported thereon, it is impractical to adapt the conventional definition for "cell size" according to JIS A 6931. For this reason, the terminology "cell size" as used in the present invention denotes a net size obtained by subtracting the thickness of a partition from the cell size according to JIS A 6931 (1978).

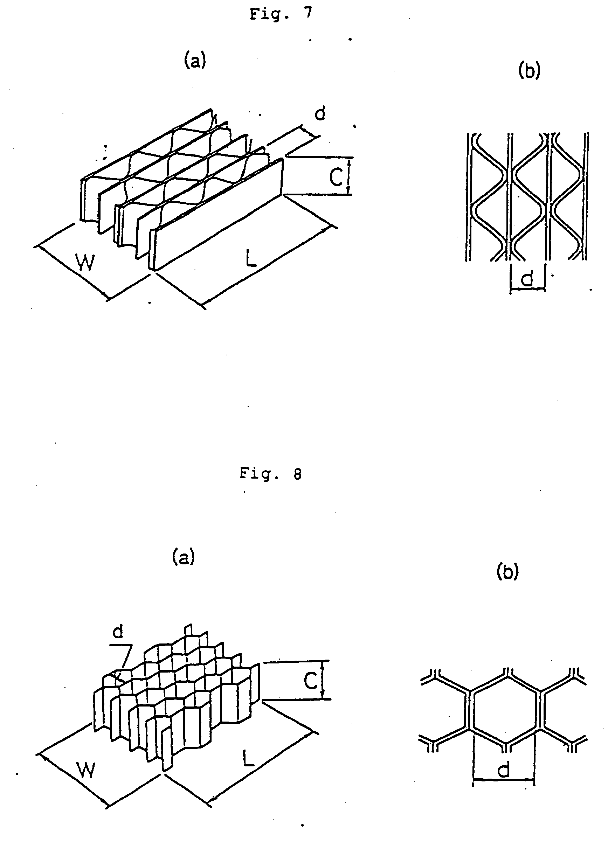

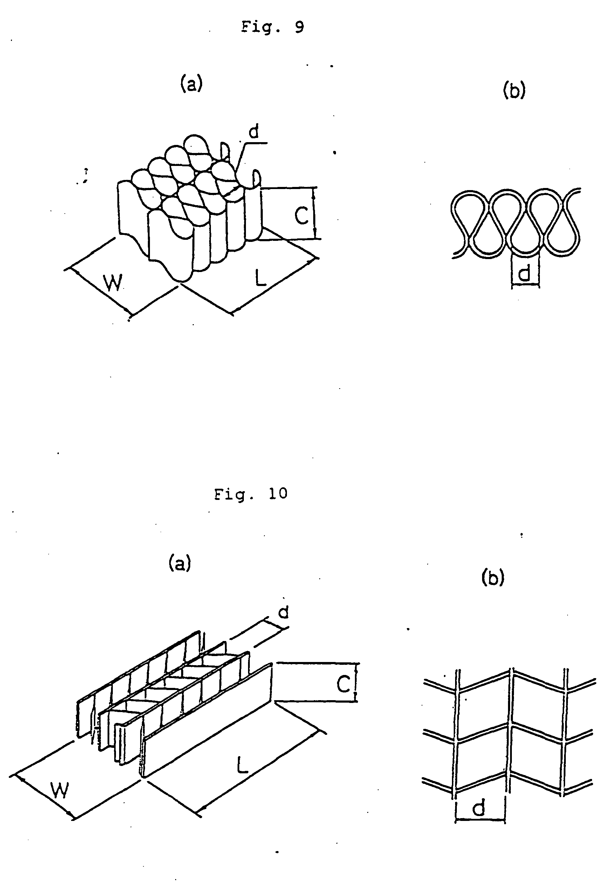

The paper cores which can be used in the present invention as a honeycomb are shown in Figs. 7 through 11, which are also quoted from JIS A 6931 (1978) with an exception that the cell size according to the definition of the invention is represented by symbol d. In each of these figures, Fig. A is a perspective view, and Fig. B is a partial plane view. Fig. 7 shows the above-described structure of corrugated cardboard type. Fig. 8 shows hexagonal cells (honeycomb cells). Fig. 9 shows circular cells made up of S-shaped partitions. Fig. 10 shows cells made up of parallel ribs.

A honeycomb having a cell size of smaller than 2 mm furnishes a large surface area for supporting a preparation but shows resistance against passage of an air flow. It tends to follow that the amount of air supplied is insufficient for securing a sufficient vaporization rate. On the other hand, a honeycomb having a cell size exceeding 5 mm has a small surface area for vaporizing the preparation so that the vaporization rate tends to be insufficient. With respect to the thickness of the partitions, it is not limited as long as a sufficient strength of the honeycomb structure is secured.

The material of the carrier is not limited to paper, and other materials as described previously can be used as well. The preparations which can be supported on the carrier includes those described previously. The preparation to be supported can contain other auxiliary components. For example, a sublimating substance can be added as a vaporization and diffusion accelerator. Where a pyrethroid compound is used as a harmful insect controlling component, a known synergist of pyrethrin is preferably used in combination. Addition of an antioxidant, e.g., 2,6-di-t-butyl-p-cresol (hereinafter abbreviated as BHT) or butylated hydroxyanisole (hereinafter abbreviated as BHA), and an ultraviolet absorber makes the preparation stable against light, heat or oxidation. A substance indicative of time passage, called a time indicator, can be added to the preparation so that the residual amount of the preparation can be visualized.

The amount of the harmful insect controlling component and/or other various components to be supported on the carrier is not particularly limited. For instance, the amount of an active ingredient, such as a harmful insect controlling component, to be supported on an oil-absorbing carrier made of, e.g., paper usually ranges from 10 to 1000 mg, preferably from 20 to 700 mg, per gram of the carrier. More specifically, where 12-hour operation per day for one month is intended, a standard amount to be supported is 2000 to 4000 mg of empenthrin on a honeycomb weighing about 10 g; 100 to 1500 mg of transfluthrin on a honeycomb weighing about 2 to 5 g; 200 to 3000 mg of terallethrin on a honeycomb weighing about 2 to 5 g; or 150 to 2500 mg of S-1295 on a honeycomb weighing about 2 to 5 g.

Addition of a time-indicating function to the carrier itself by using, for example, a colorant that changes its color with time brings about convenience in practical use. Time indicators which can be used for this purpose include a water-and alcohol-soluble base or acid indicator combined with, as an assistant, a water-soluble basic or acidic agent, a color changing system comprising a basic indicator and an alkaline substance, and a time indicating system comprising an electron-donating organic color developer modified with a low-molecular organic modifier and a volatile desensitizer. The time indicating system comprising a low-molecular organic modifier-modified electron-donating organic color developer and a volatile desensitizer is preferred for its usefulness.

An air flow is blown to the above-described preparation-on-carrier in the direction through the cells to vaporize and diffuse the preparation. If the air flow is less than 0.1 1/sec·m², it is difficult to assure a sufficient rate of vaporization. If it exceeds 10 l/sec·m², impractical high power is required. With the case of the apparatus 1 shown in Fig. 1, the air flow at the air outlet ranges from 0.2 to 6 l/sec.

The following tests were carried out to examine the performance of the carrier according to Example 3.

### Test 6:

A carrier 10a shown in Fig. 2 (70 x 70 x 5 mm) was prepared using a laminate of single-sided corrugated cardboard made of bleached kraft paper. The basis weight of the liner tape and the fluted tape per unit length was 70 g/m and 120 g/m, respectively. Six carriers 10a were prepared with the cell size varied from 1.7 mm to 10 mm. Each carrier 10a was uniformly impregnated with a preparation containing 1 g of transfluthrin.

The impregnated carrier, i.e., preparation-on-carrier 10 was set in the harmful insect controlling apparatus 1 shown in Fig. 1. The fan 8 was operated for 30 minutes by the direct current motor 5 to blow air to vaporize transfluthrin. The vaporized transfluthrin was trapped in a silica gel trap according to the method described below and quantitatively analyzed by gas chromatography. The direct current motor 5 used has a power consumption of 100 mA or less with no load applied and runs for a long time on two size-D manganese dry batteries. The fan 8 used produces an air flow of 2 l/sec when driven by the direct current motor 5 on two size-D manganese dry batteries (3 V).

The above test was repeated in the same manner except for driving the fan 8 at a voltage of 1.5 V.

### Method for Analyzing Vaporized Component:

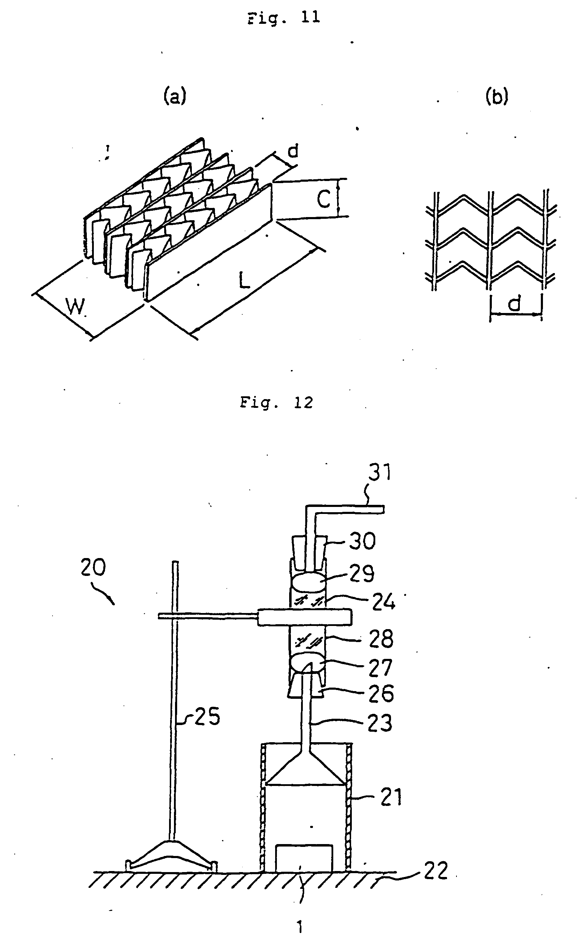

Fig. 12 illustrates a silica gel trap 20 used for trapping the vaporized transfluthrin. An open-ended cylinder was put on a horizontal mount 22, and the apparatus 1 was placed therein. The open top of the cylinder 21 was closed with an inverted funnel. An open-ended glass tube 24 having an inner diameter of 43 mm and a length of 140 mm was set vertically in the air above the cylinder 21 by means of a stand 25. Into the lower open end of the glass tube 24 was fitted the tip of the funnel 23 via a rubber stopper 26, and the tip of the funnel projecting over the rubber stopper 26 was covered with a cotton wad 27. The glass tube 24 was filled with 40 g of silica gel 28. The top of the silica gel was covered with a cotton wad 29, and a ventilating tube 31 was fitted into the upper open end of the glass tube 24 via a rubber stopper 30. The ventilating tube 31 was connected to a vacuum pump (not shown).

The apparatus 1 was operated to vaporize transfluthrin within the cylinder 21. The cylinder 21 and the glass tube 24 were evacuated by means of the vacuum pump to let transfluthrin vapor be adsorbed onto the silica gel 28.

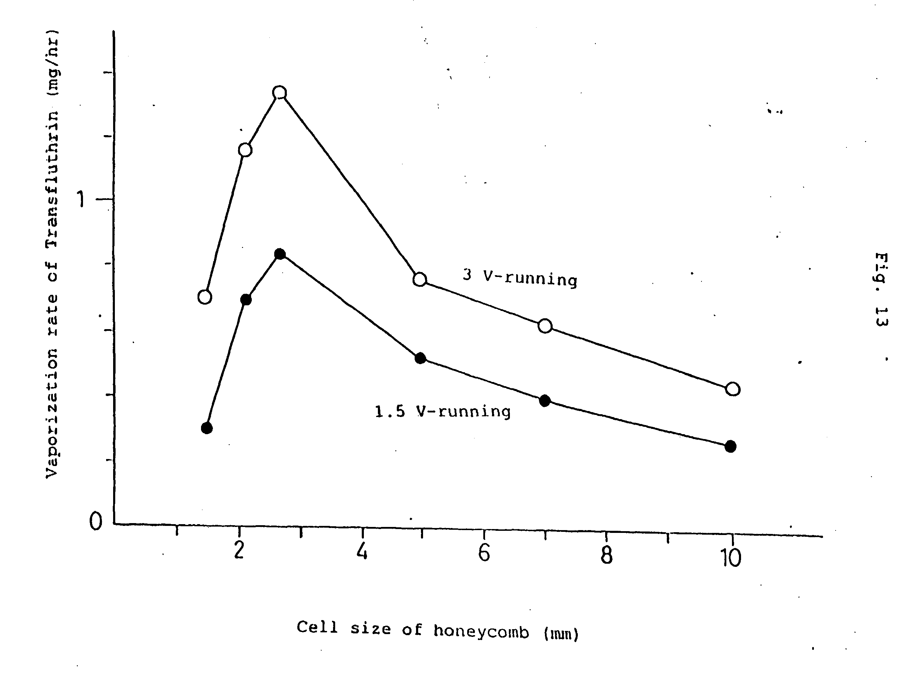

The amount of the vaporized transfluthrin (mg/hr) thus determined was plotted against the cell size of the honeycomb carrier 10a. The plots are depicted in Fig. 13, in which hollow circles and solid circles show the results of 3 V driving and 1.5 V driving, respectively. It is seen that the amount of vaporized transfluthrin per unit time is large with the cell size ranging from 2 to 5 mm, particularly from 2 to 3.5 mm.

### Test 7 (Controlling Effect on Culex pipiens pallens):

Three honeycomb carriers of Fig. 2 (35 x 35 x 15 mm) having a cell size of 2.2 mm (sample No. 1), 2.7 mm (sample No. 2) and 5.0 mm (sample No. 3), respectively, were prepared and impregnated with a preparation containing 1 g of transfluthrin. Each of the resulting preparation-on-carriers was set in the harmful insect controlling apparatus of Fig. 1 which had an air flow of 1 l/sec. For comparison, a commercially available liquid type electric mosquito controlling apparatus containing allethrin as an active ingredient was used.

Testing was conducted in a room of 2.7 m by 3.6 m. Four cages each containing 20 to 25 female imagoes of *Culex pipiens pallens* were hung from the ceiling; two cages were 150 cm high from the floor, and other two 75 cm high from the floor. The apparatus was operated in the room for 2 hours, and the number of knocked down insects was counted for every 10 minutes. After the 2-hour testing, the insects were transferred into a plastic container. After 24 hours from the start of testing, the number of dead insects was counted to obtain the death rate. The results obtained are shown in Table 2.

**TABLE 2**

| Effect on *Culex pipiens pallens* | | | |
|---|---|---|---|
| Sample | KT₅₀ (min) | KT₉₀ (min) | Death Rate (%) |
| No. 1 | 46 | 56 | 78.9 |
| No. 2 | 40 | 47 | 91.5 |
| No. 3 | 67 | 90 | 66.7 |
| Comparison | 55 | 82 | 65.1 |

### FORMULATION EXAMPLE

### Preparation-on-carrier

### Formulation 1:

A ceramic honeycomb of 70 x 70 x 15 mm impregnated with a preparation comprising 4 g of empenthrin and 0.1 g of BHT, for 12-hour use per day for 30 days.

### Formulation 2:

A paper honeycomb of 60 x 50 x 5 mm impregnated with a preparation comprising 0.5 g of transfluthrin, 0.05 g of BHT, and 0.2 g of isopropyl myristate, for 12-hour use per day for 30 days. Formulation 3:

A paper honeycomb of 60 x 60 x 5 mm impregnated with a preparation comprising 0.5 g of S-1295 and 0.05 g of BHT; for 12-hour use per day for 30 days.

### Formulation 4:

A paper honeycomb of 70 x 70 x 10 mm impregnated with a preparation comprising 1 g of terallethrin and 0.05 g of BHT; for 12-hour use per day for 30 days.

### Liquid Preparation

| Formulation 5: | |
|---|---|
| Empenthrin | 5.0 g |
| 2,6-di-butylhydroxytoluene | 0.6 g |
| Perfume | 0.1 g |
| Kerosine | 35 ml |

| Formulation 6: | |
|---|---|
| Benfluthrin | 0.6 g |
| 2,6-di-butylhydroxytoluene | 0.1 g |
| Perfume | 0.1 g |
| Isopropyl myristate | 8 ml |
| Kerosine | 32 ml |

| Formulation 7: | |
|---|---|
| Prallethrin | 1.3 g |
| 2,6-di-butylhydroxytoluene | 0.1 g |
| Perfume | 0.1 g |
| Kerosine | 40 ml |

Water-based Preparation

| Formulation 8: | |
|---|---|
| Benfluthrin | 0.6 g |
| Butyl carbitol | 25 ml |
| Water | 25 ml |
| Butylhydroxytoluene | 0.20 g |

| Formulation 9: | |
|---|---|
| Empenthrin | 2.0 g |
| Butyl carbitol | 25 ml |
| Propylene glycol . | 17 ml |
| Water | 8 ml |
| Butylhydroxytoluene | 0.20 g |

According to Example 3 in which a preparation is supported on a honeycomb carrier having a great number of through cells having a cell size of 2 to 5 mm, the active ingredient is vaporized at a high rate by air blowing. Where a perfume component is used in combination with the active ingredient, the preparation-on-carrier diffuses an aroma.

### EXAMPLE 4

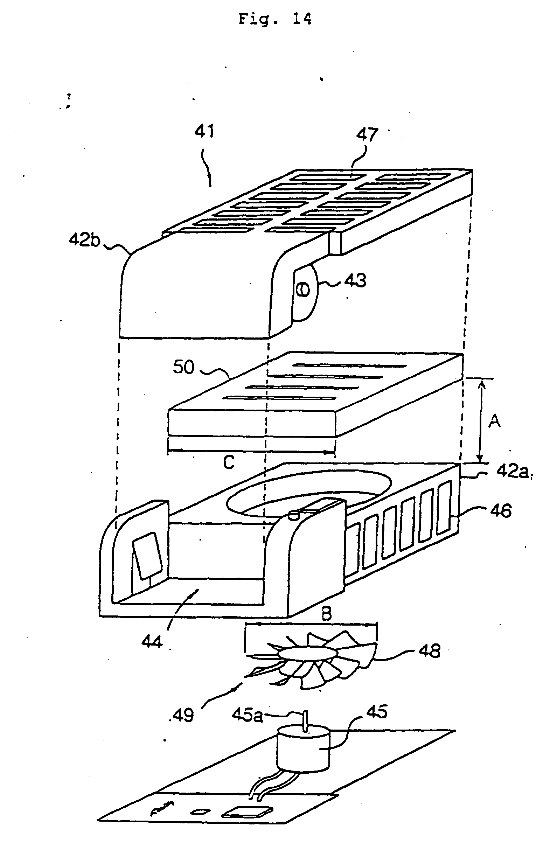

Fig. 14 is an exploded view of a harmful insect controlling apparatus 41. The apparatus 41 comprises a chamber 42a, 42b, a preparation-on-carrier 50 that is put in the chamber, and a blowing means 49. An air current from the blowing means 49 is applied to the preparation-on-carrier 50 to vaporize a harmful insect controlling component of the preparation and to diffuse the vapor outside the chamber 42b.

The blowing mechanism 49 is composed of a drive motor 45 and a fan 48 driven by the motor 45. The fan 48 sucks air through an air inlet 46 of the chamber 42a to generate an upward air flow across the chamber 42a against the preparation-on-carrier 50. The air flow is discharged out of the chamber 42b through an air outlet 47, carrying the vapor of the harmful insect controlling component.

The diameter B of the fan 48 is smaller than the length C of the preparation-on-carrier 50 in the direction perpendicular to the air flow direction. The distance A between the fan 48 and the preparation-on-carrier 50 in the air flow direction is limited within a predetermined range. The distance A in Example 4 is set at 5 mm.

The motor 45 is driven on a dry battery 43 put in a battery box 44 to revolve a drive shaft 45a and to operate the fan 48 fixed on the drive shaft 45a.

The chamber 42a or 42b may be provided with a fastener for fastening the lower part of the preparation-on-carrier 50. The fastener is not limited in shape and includes a support, a projection, and a net. Instead of the fastener, an air flow regulating plate may be placed beneath the preparation-on-carrier 50 to support the preparation-on-carrier 50. In these cases, the distance A means the distance between the fan 48 and the fastener or the air flow regulating plate.

In order to examine the influence of the distance A on the air flow (at the air outlet 47) and the vaporization rate of the harmful insect controlling component, the following test was carried out. Test 8:

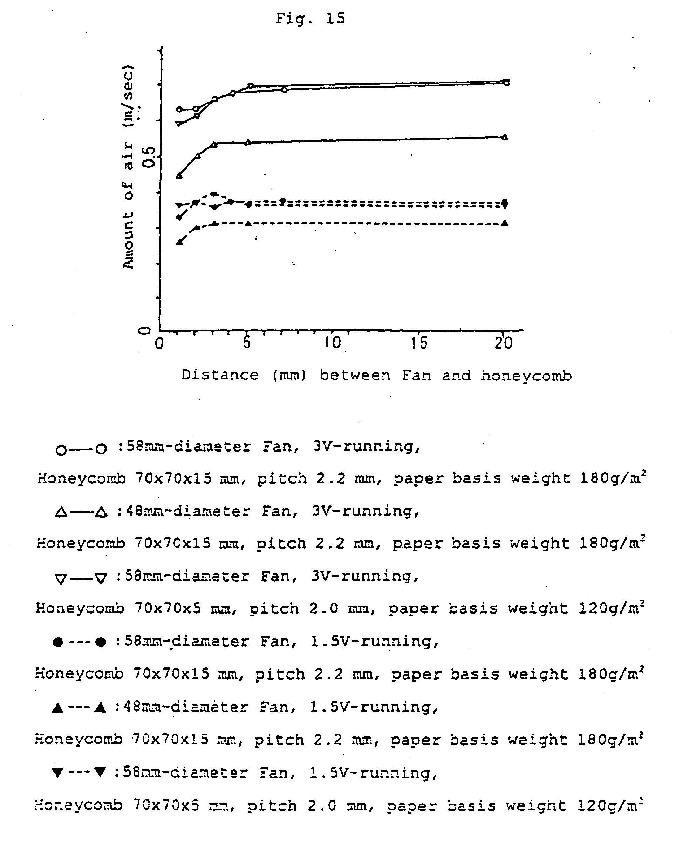

The apparatus 41 was assembled using a propeller fan manufactured by Shiko Giken and a honeycomb as a carrier with a varied distance A (the distance between the fan and the carrier). The apparatus was operated at a driving voltage of 1.5 V or 3.0 V. Changes in air flow with changes of the distance A were measured, and the results obtained are shown in Fig. 15 to 17. The particulars of the propeller fan and the honeycomb were as follows. The combinations of the testing conditions, i.e., the propeller fan, the honeycomb, and the driving voltage are described in Figs. 15 through 17.
Propeller fan 1: diameter: 58 mm; 11-bladed
Propeller fan 2: diameter: 48 mm; 11-bladed
   - Honeycomb 1:: 70 x 70 x 15 mm; cell size: 2.2 mm; basis weight: 180 g/m²
   - Honeycomb 2:: 70 x 70 x 5 mm; cell size: 2.0 mm; basis weight: 120 g/m²

Fig. 15 is a graph showing changes in air flow with changes of distance A. Every line of the graph shows little variation in air flow in the distance A range of from 5 to 20 mm. On the other hand, with the distance A being smaller than 5 mm, every line displays a slight decrease of air flow.

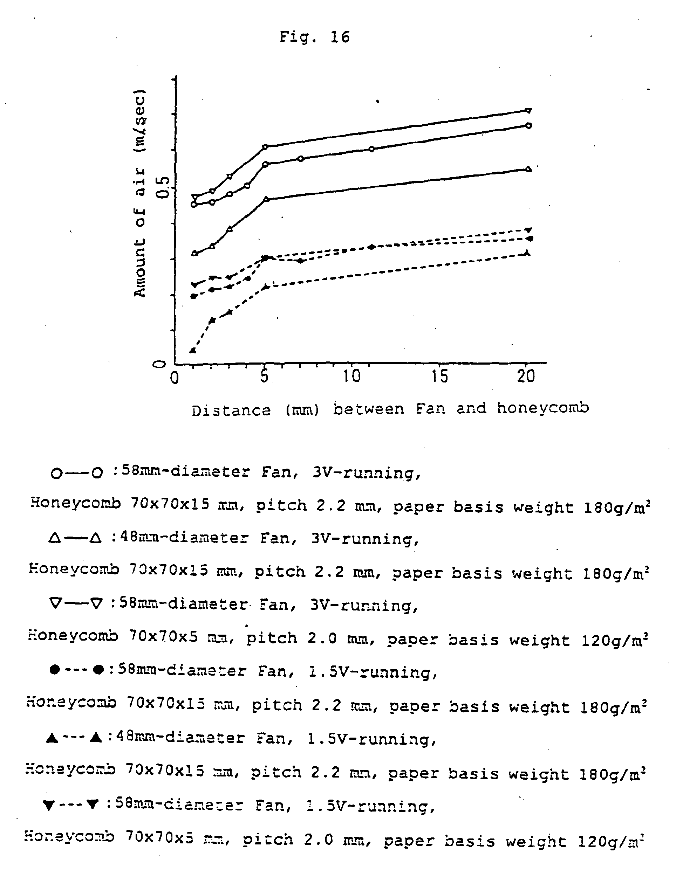

Fig. 16 shows the results obtained in the same manner as described above, except for using a harmful insect controlling apparatus equipped with a suction type fan in place of the pressure fan used in Fig. 14.

Comparing Fig. 15 with Fig. 16, the decrease of air flow (distance A<5 mm) in Fig. 15 is smaller than that observed in Fig. 16. In Fig. 16, every line of the graph shows a gradual decrease in air flow as the distance A reduces from 20 mm to 5 mm and a drastic decrease as the distance A further reduces from 5 mm.

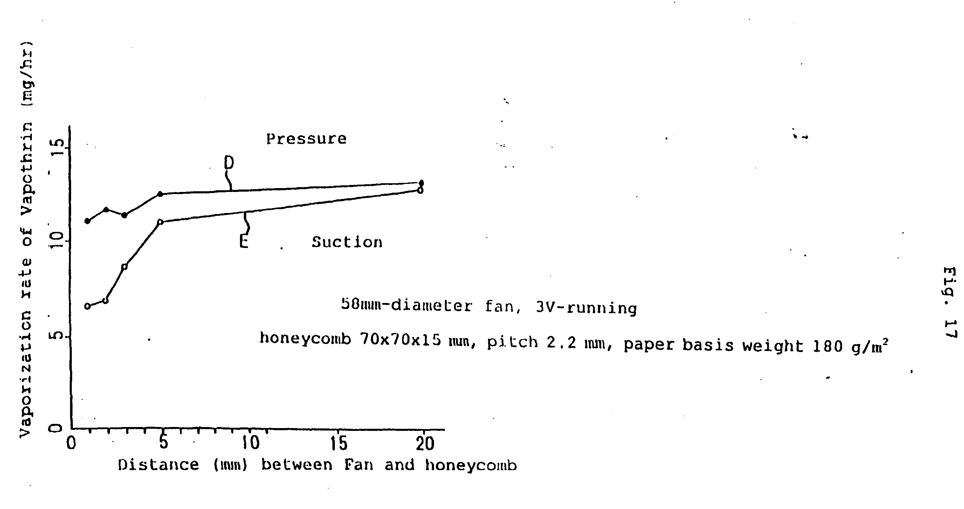

Fig. 17 shows changes in vaporization rate of the harmful insect controlling component against changes in distance A in the harmful insect controlling apparatus of Fig. 14 equipped with the pressure fan and the apparatus equipped with the suction fan. Lines D and E represent the results of the apparatus of Fig. 14 and the apparatus with the suction fan, respectively. While the vaporization rate decreases gradually as the distance A decreases from 20 mm to 5 mm in both lines D and E, the degree or decrease in line D is fairly smaller than that observed with line E. As the distance A further decreases, line E displays a sudden fall of the vaporization rate. The fall is still greater than the decrease in air flow shown in Fig. 16. On the other hand, line D shows a lower vaporization rate with a distance A shorter than 5 mm than that with a distance A of from 5 to 20 mm, but this decrease is far smaller than that observed with line E.

From these results, it can be understood that both suppression of a decrease in vaporization rate and size reduction of an apparatus, which are conflicting to each other, can be accomplished by (1) using a pressure fan (fan 48), (2) making the diameter of the fan 48 smaller than the side length of the preparation-on-carrier 50 (the side being perpendicular to the air flow direction), and (3) setting the fan 48 and the preparation-on-carrier 50 at a distance of about 5 mm.

In short, the apparatus of Example 4 is of pressure type having the preparation-on-carrier 50 between the fan 48 and the air outlet 47, and the fan 48 and the preparation-on-carrier 50 are set at a prescribed distance (about 5 mm), and the diameter of the fan 48 is smaller than the side length of the preparation-on-carrier 50 (the side being perpendicular to the air flow direction). Therefore, reduction in performance, such as a reduction in vaporization rate of the harmful insect controlling component, can be minimized to secure a sufficient amount of vapor of the component while achieving size reduction of the apparatus. T

### EXAMPLE 5

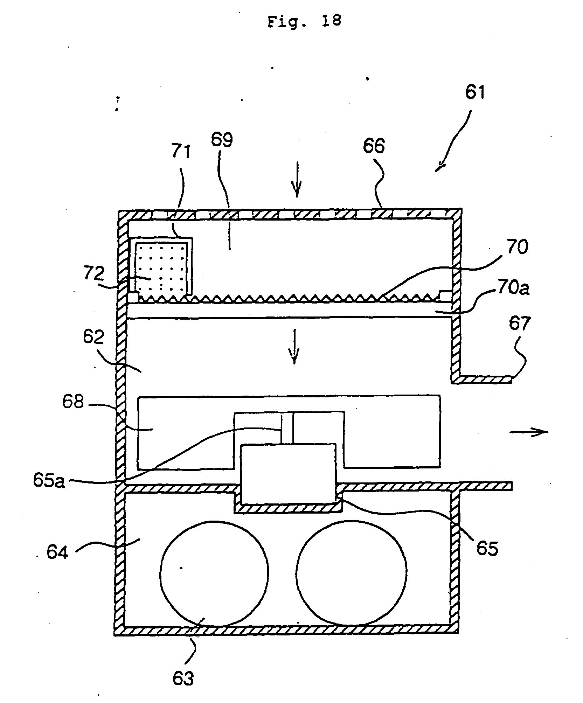

Fig. 18 schematically illustrates a harmful insect controlling apparatus 61, which comprises a chamber 69 in which a net 70 is set as a carrier for supporting a preparation, being supported on a supporting frame 70a, a chamber 62 having a direct current motor 65 and a fan 68, and a battery box 64 having batteries 63.

Since the capacity of the net 70 to hold a preparation is not so great, the preparation initially supported on the net will be exhausted soon. It is therefore necessary to continuously feed the preparation to the net 70. For this purpose, a preparation feed box 71 which is made of a preparation-impermeable material and filled with preparation-containing gel is provided on the end of the net 70 with its bottom open so that the preparation oozes out of the gel 72 and spreads throughout the net 70.

The preparation-containing gel 72 is prepared by using a gelatinizer. Preferred of known gelatinizers are metal soaps, fatty acids or salts thereof, metal salts of long-chain alkyl phosphates, hydroxy acids, POE alcohol ethers, fatty acid esters, glycerol fatty acid esters, and fatty acid ethanolamines.

The gel 72 may be relatively hard gel having a high viscosity, from which a liquid preparation oozes and gradually spreads throughout the carrier, or it may be soft gel having a low viscosity, from which a preparation flows to the carrier as a viscous liquid. In the latter case, the gelatinizer also spreads over the carrier to broaden the surface area of the carrier or to increase the capacity of the carrier to hold the preparation. In particular, the net used in Example 5 as a carrier can enjoy the advantage that the surface area for evaporating the preparation is broadened.

The active ingredient of the preparation fed to and supported by the carrier is not particularly limited as long as it evaporates into the air to produce a certain effect, such as an insecticidal effect or an insect repellent effect. Useful active ingredients include the harmful insect controlling component used in Example 1 and the aromatics used in Example 3. The content of the active ingredient such as a harmful insect controlling component in the gel is not particularly limited and usually ranges from 2 to 500 mg/g, preferably from 5 to 100 mg/g.

The amount of the preparation to be fed to the net per day, in terms of amount of the active ingredient, is typically as follows. When a net of 60 mm x 60 mm is used, and the apparatus is to be operated for 12 hours a day for 1 month, the amount of transfluthrin, terallethrin or S-1295 to be fed to the net is 30 to 360 mg/net, 50 to 750 mg/net or 40 to 500 mg/net, respectively.

It is preferred for the preparation to contain a colorant and the like so that the residual amount of the preparation may be seen visually. The colorant is preferably such that is soluble in the solvent of the preparation and migrates in the gel together with the preparation. Such colorants include 3,3-bis(1-n-butyl-2-methylindol-3-yl)phthalide, 3-(4-diethylamino-2-ethoxyphenyl)-3-(1-ethyl-2-methylindol-3-yl)-4-azaphthalide, and 1,3-dimethyl-6-diethylaminofluoran.

An air flow is blown across the above-described net carrier at a substantial flow rate of 0.1 to 10 1/sec·m² to vaporize and diffuse the preparation. If the flow rate is less than 0.1 /sec·m², it is difficult to assure a sufficient vaporization rate. If it exceeds 10 1/sec·m², high power is required, which is unfavorable economically.

In Fig. 18, the chamber 62 under the chamber 69 is equipped with the direct current motor 65, and the fan 68 is fixed to the tip of a drive shaft 65a of the motor. The chamber 62 has an air outlet 67 on its side. The motor 65 is driven on the batteries 63 to revolve the fan 68, whereby the air is inhaled through an air inlet of the chamber 69 and passed across the net 70 to vaporize the supported preparation. The air containing the vapor of the preparation is sent forth out of the apparatus 61 through the outlet 67 by the fan 68 to carry out harmful insect control.

According to Example 5, an effective amount of the active ingredient is vaporized at a small vaporization rate for an extended period of time; the vaporization rate is easily adjustable; the preparation is fed continuously to the carrier; and exhaustion of the preparation is recognized clearly and objectively by any user.

### EXAMPLE 6

Fig. 19 shows an example of a carrier with which a means for feeding a preparation is combined. The means for feeding a preparation is a doughnut-shaped container 91 filled with a liquid preparation 92. The liquid preparation 92 can be gel or sol. The liquid preparation 92 is poured into the bottle 91 through an inlet 93 that is made on the upper side of the bottle. The bottle 91 also has openings 94 through which the end of a carrier is inserted in.

A rectangular absorbent member 90, which is a carrier for supporting the preparation, is set with its both ends 96 inserted into the bottle through the openings 94 and soaked in the liquid preparation 92 thereby to feed the preparation to its central portion continuously. The central portion 95 of the absorbent member 90 can have a honeycomb structure.

Air is blown to the rectangular absorbent member 90 as indicated by the arrow to vaporize the preparation supported by the absorbent member 90. Since the liquid preparation can be fed to an amount exceeding the saturated absorbing capacity of the absorbent member 90, vaporization continues for a prolonged period of time. A part or the whole of the bottle 91 can be made of a transparent or semi-transparent material, whereby the residual amount of the preparation 92 can be seen with the eye.

## Claims

1. A harmful insect controlling apparatus (1) comprising
(a) a chamber (2) having an air inlet (6) and an air outlet (7),
(b) a blowing means which is set in the chamber and comprises a fan (8) which has a weight of less than 30 g causing a flow of air from the air inlet (6) to the air outlet (7),
(c) a preparation-on-carrier (10) which is or is to be set in the flow of air and comprises a carrier having supported thereon a harmful insect controlling preparation comprising a harmful insect controlling compound which is sparingly volatile at ordinary temperature, and
(d) a means for driving the fan which comprises a direct current motor (5) consuming a current of 25 mA or lower with no load applied at a voltage of 3 V,
(e) a dry battery power source for the motor (5), such that in use said motor (5) drives said fan (8) to provide a flow of air to said preparation-on-carrier (10) to vaporize and diffuse said harmful insect controlling compound, and so that in use said air flow is 0.2 to 6 1/sec at said air outlet (7).

2. The harmful insect controlling apparatus according to claim 1, wherein the fan (8) has a plurality of blades extending radially from the axis of its rotation.

3. A harmful insect controlling apparatus according to claim 1 or 2, further comprising a means for regulating the flow of air so as to optimize the vaporization rate of the active ingredient for the conditions of use.

4. A harmful insect controlling apparatus according to claim 3, wherein said regulating means is said carrier (10) set in the flow of air or a separate flow regulator.

5. A harmful insect controlling apparatus according to any one of claims 1 to 4, wherein said preparation-on-carrier (10) is a carrier having supported thereon the preparation, and said carrier has a honeycomb shape, a net shape, a slit shape, a lattice shape or a shape as perforated paper, having a plurality of vent holes which are open to the flow of air in the chamber and arrayed in a plane perpendicular to the flow of air, and is prepared from an organic or inorganic molding material capable of holding the preparation.

6. A harmful insect controlling apparatus according to any one of claims 1 to 5, wherein said apparatus (61) has a means (71) for supplying the preparation (72) to said carrier (70), and said means has a gelling material containing said preparation.

7. A harmful insect controlling apparatus according to any one of claims 1 to 6, wherein the carrier has a net structure (70) or a honeycomb structure (10a).

8. A harmful insect controlling apparatus according to claim 7, wherein said preparation-on-carrier (10) is a honeycomb having supported thereon said preparation, said honeycomb having a large number of through cells having a cell size of 2 to 5 mm.

9. A harmful insect controlling apparatus according to claim 8, wherein said honeycomb has 200 to 2500 through cells.

10. A method of controlling harmful insects which comprises providing an apparatus (1) comprising
(a) a chamber (2) having an air inlet (6) and an air outlet
(b) a blowing means which is set in the chamber and comprises a fan (8) which has a weight of less than 30g causing a flow of air from the air inlet (6) to the air outlet (7),
(c) a preparation-on-carrier (10) which is set in the flow of air and comprises a carrier having supported thereon a harmful insect controlling preparation comprising a harmful insect controlling compound which is sparingly volatile at ordinary temperature, and
(d) a means for driving the fan which comprises a direct current motor (5) consuming a current of 25 mA or lower with no load applied at a voltage of 3 V,
(e) a dry battery power source for the motor (5) , and driving said fan (8) by said motor (5) to provide a flow of air to said preparation-on-carrier (10) to vaporize and diffuse said harmful insect controlling compound, and so that said air flow is 0.2 to 6 l/sec at said air outlet (7).

11. A method according to claim 10, further comprising providing a means for regulating the flow of air so as to optimize the vaporization rate of the active ingredient for the condition of use.

12. A method according to claim 11, wherein said regulating means is said carrier (10) set in the flow of air or a separate flow regulator.

13. a method according to any one of claims 10 to 12, wherein said preparation-on-carrier (10) is a carrier having supported thereon the preparation, and said carrier has a honeycomb shape, a net shape, a slit shape, a lattice shape or a shape such as perforated paper, having a plurality of vent holes which are open to the flow of air in the chamber and arrayed in the plane perpendicular to the flow of air and is prepared from an organic or inorganic molding material capable of holding the preparation.

14. A method according to any one of claims 10 to 13, wherein said apparatus (61) has a means (71) for supplying the preparation (72) to said carrier (70), and said means has a gelling material containing said preparation

15. A method according to any one of claims 10 to 14, wherein the carrier has a net structure (70) or a honeycomb structure (10a).

16. A method according to claim 15, wherein said preparation-on-carrier (10) is a honeycomb having supported thereon said preparation, said honeycomb having alarge number of through cells having a cell size of 2 to 5 mm.

17. A method according to claim 16, wherein said honeycomb has 200 to 2500 through cells.

## Patentansprüche

1. Vorrichtung (1) zum Bekämpfen von Schadinsekten, mit:
(a) einer Kammer (2) mit einem Lufteinlass (6) und einem Luftauslass (7);
(b) einer Gebläseeinrichtung, die in die Kammer eingesetzt ist und über einen Lüfter (8) mit einem Gewicht unter 30 g verfügt, der für eine Luftströmung vom Lufteinlass (6) zum Luftauslass (7) sorgt;
(c) einem Präparatträger (10), der sich in der Luftströmung befindet oder in diese einzusetzen ist, und über einen Träger verfügt, der ein Präparat zum Bekämpfen von Schadinsekten mit einer bei normaler Temperatur kaum flüchtigen Verbindung zum Bekämpfen von Schadinsekten trägt; und
(d) einer Einrichtung zum Antreiben des Lüfters, die über einen Gleichstrommotor (5) verfügt, der einen Strom von 25 mA oder weniger verbraucht, wenn im lastfreien Zustand eine Spannung von 3 V angelegt ist;
(e) einer Trockenbatterie-Spannungsquelle für den Motor (5), so dass dieser im Betrieb den Lüfter (8) antreibt, um für eine Luftströmung zum Präparatträger (10) zu sorgen, um die Verbindung zum Bekämpfen von Schadinsekten zu verdampfen und zu verteilen, und so dass im Gebrauch die Luftströmung am Luftauslass (7) 0,2 bis 6 l/s beträgt.

2. Vorrichtung zum Bekämpfen von Schadinsekten nach Anspruch 1, bei der der Lüfter (8) über mehrere Blätter verfügt, die sich radial ausgehend von seiner Rotationsachse erstrecken.

3. Vorrichtung zum Bekämpfen von Schadinsekten nach Anspruch 1 oder 2, ferner mit einer Einrichtung zum Regulieren der Luftströmung zum Optimieren der Verdampfungsrate des aktiven Bestandteils für die Anwendungsbedingungen.

4. Vorrichtung zum Bekämpfen von Schadinsekten nach Anspruch 3, bei der die Reguliereinrichtung der in die Luftströmung eingesetzte Träger (10) oder ein separater Flussregler ist.

5. Vorrichtung zum Bekämpfen von Schadinsekten nach einem der Ansprüche 1 bis 4, bei der der Präparatträger (10) ein Träger ist, der das Präparat trägt, und er über Wabenform, Netzform, Schlitzform, Gitterform oder eine Form wie perforiertes Papier verfügt, mit einer Anzahl von Belüftungslöchern, die für die Luftströmung in der Kammer offen sind und die in einer Ebene orthogonal zur Luftströmung angeordnet sind, wobei er aus einem organischen oder anorganischen Formungsmaterial hergestellt ist, das das Präparat halten kann.

6. Vorrichtung zum Bekämpfen von Schadinsekten nach einem der Ansprüche 1 bis 5, wobei diese Vorrichtung (61) über eine Einrichtung (71) zum Zuführen des Präparats (72) zum Träger (70) verfügt, die ein das Präparat enthaltendes Geliermittel aufweist.

7. Vorrichtung zum Bekämpfen von Schadinsekten nach einem der Ansprüche 1 bis 6, bei der der Träger über eine Netzstruktur (70) oder eine Wabenstruktur (10a) verfügt.

8. Vorrichtung zum Bekämpfen von Schadinsekten nach Anspruch 7, bei der der Präparatträger (10) eine Wabe ist, die das Präparat trägt und die über eine große Anzahl von durchgehenden Zellen mit einer Zellengröße von 2 bis 5 mm verfügt.

9. Vorrichtung zum Bekämpfen von Schadinsekten nach Anspruch 8, bei der die Wabe über 200 bis 2500 durchgehende Zellen verfügt.

10. Verfahren zum Bekämpfen von Schadinsekten, zu dem ferner Folgendes gehören:
Bereitstellen einer Vorrichtung (1) mit:
(a) einer Kammer (2) mit einem Lufteinlass (6) und einem Luftauslass (7);
(b) einer Gebläseeinrichtung, die in die Kammer eingesetzt ist und über einen Lüfter (8) mit einem Gewicht unter 30 g verfügt, der für eine Luftströmung vom Lufteinlass (6) zum Luftauslass (7) sorgt;
(c) einem Präparatträger (10), der sich in der Luftströmung befindet, und über einen Träger verfügt, der ein Präparat zum Bekämpfen von Schadinsekten mit einer bei normaler Temperatur kaum flüchtigen Verbindung zum Bekämpfen von Schadinsekten trägt; und
(d) einer Einrichtung zum Antreiben des Lüfters, die über einen Gleichstrommotor (5) verfügt, der einen Strom von 25 mA oder weniger verbraucht, wenn im lastfreien Zustand eine Spannung von 3 V angelegt ist;
(e) einer Trockenbatterie-Spannungsquelle für den Motor (5);
und Antreiben des Lüfters (8) durch den Motor (5), um für eine Luftströmung zum Präparatträger (10) zu sorgen, um die Verbindung zum Bekämpfen von Schadinsekten zu verdampfen und zu verteilen, und so, dass im Gebrauch die Luftströmung am Luftauslass (7) 0,2 bis 6 l/s beträgt.

11. Verfahren nach Anspruch 10, ferner mit einer Einrichtung zum Regulieren der Luftströmung zum Optimieren der Verdampfungsrate des aktiven Bestandteils für die Anwendungsbedingungen.

12. Verfahren nach Anspruch 11, bei dem die Reguliereinrichtung der in die Luftströmung eingesetzte Träger (10) oder ein separater Flussregler ist.

13. Verfahren nach einem der Ansprüche 10 bis 12, bei dem der Präparatträger (10) ein Träger ist, der das Präparat trägt, und er über Wabenform, Netzform, Schlitzform, Gitterform oder eine Form wie perforiertes Papier verfügt, mit einer Anzahl von Belüftungslöchern, die für die Luftströmung in der Kammer offen sind und die in einer Ebene orthogonal zur Luftströmung angeordnet sind, wobei er aus einem organischen oder anorganischen Formungsmaterial hergestellt ist, das das Präparat halten kann.

14. Verfahren nach einem der Ansprüche 10 bis 13, wobei diese Vorrichtung (61) über eine Einrichtung (71) zum Zuführen des Präparats (72) zum Träger (70) verfügt, die ein das Präparat enthaltendes Geliermittel aufweist.

15. Verfahren nach einem der Ansprüche 10 bis 14, bei dem der Träger über eine Netzstruktur (70) oder eine Wabenstruktur (10a) verfügt.

16. Verfahren nach Anspruch 15, bei dem der Präparatträger (10) eine Wabe ist, die das Präparat trägt und die über eine große Anzahl von durchgehenden Zellen mit einer Zellengröße von 2 bis 5 mm verfügt.

17. Verfahren nach Anspruch 16, bei dem die Wabe über 200 bis 2500 durchgehende Zellen verfügt.

## Revendications

1. Appareil de contrôle des insectes nuisibles (1) comprenant
(a) une chambre (2) ayant une entrée d'air (6) et une sortie d'air (7),
(b) un moyen de souffleur qui est placé dans la chambre et comprend un ventilateur (8) qui a un poids de moins de 30 g provoquant un écoulement d'air depuis l'entrée d'air (6) vers la sortie d'air (7),
(c) une préparation sur le transporteur (10) qui est ou doit être placée dans l'écoulement d'air et comprend un transporteur ayant supporté sur lui une préparation de contrôle des insectes nuisibles comprenant un composé de contrôle des insectes nuisibles qui est modérément volatil à température ordinaire, et
(d) un moyen pour entraîner le ventilateur qui comprend un moteur à courant continu (5) consommant un courant de 25 mA ou moins sans charge appliquée à une tension de 3 V,
(e) une source de courant à batterie sèche pour le moteur (5), de telle sorte que en utilisation ledit moteur (5) entraîne ledit ventilateur (8) pour produire un écoulement d'air vers ladite préparation sur le transporteur (10) pour vaporiser et diffuser ledit composé de contrôle des insectes nuisibles et de telle sorte que en utilisation ledit écoulement d'air est de 0,2 à 6 1/sec au niveau de ladite sortie d'air (7).

2. Appareil de contrôle des insectes nuisibles selon la revendication 1, dans lequel le ventilateur (8) a une pluralité de pales s'étendant de manière radiale depuis l'axe de sa rotation.

3. Appareil de contrôle des insectes nuisibles selon la revendication 1 ou 2, comprenant en outre un moyen pour réguler l'écoulement d'air de telle manière à optimiser le taux de vaporisation de l'ingrédient actif pour la condition d'utilisation.

4. Appareil de contrôle des insectes nuisibles selon la revendication 3, dans lequel ledit moyen de régulation est ledit transporteur (10) placé dans l'écoulement d'air ou un régulateur d'écoulement séparé.

5. Appareil de contrôle des insectes nuisibles selon l'une quelconque des revendications 1 à 4, dans lequel ladite préparation sur le transporteur (10) est un transporteur ayant supporté sur lui la préparation et ledit transporteur a une forme de nid d'abeille, une forme de filet, une forme de fente, une forme de treillis ou une forme comme du papier perforé, ayant une pluralité de trous de ventilation qui sont ouverts à l'écoulement d'air dans la chambre et agencés en réseau dans un plan perpendiculaire à l'écoulement d'air et est préparé à partir d'un matériau de moulage organique ou inorganique capable de supporter la préparation.

6. Appareil de contrôle des insectes nuisibles selon l'une quelconque des revendications 1 à 5, dans lequel ledit appareil (61) a un moyen (71) pour alimenter la préparation (72) audit transporteur (70) et ledit moyen a un matériau de gélification contenant ladite préparation.

7. Appareil de contrôle des insectes nuisibles selon l'une quelconque des revendications 1 à 6, dans lequel le transporteur a une structure de filet (70) ou une structure de nid d'abeille (10a).

8. Appareil de contrôle des insectes nuisibles selon la revendication 7, dans lequel ladite préparation sur le transporteur (10) est un nid d'abeille ayant supporté sur lui ladite préparation ledit nid d'abeille ayant un grand nombre de cellules traversantes ayant une taille de cellule de 2 à 5 mm.

9. Appareil de contrôle des insectes nuisibles selon la revendication 8, dans lequel ledit nid d'abeille à 200 à 2500 cellules traversantes.

10. Procédé de contrôle des insectes nuisibles qui comprend la production d'un appareil (1) comprenant
(a) une chambre (2) ayant une entrée d'air (6) et une sortie d'air (7),
(b) un moyen de souffleur qui est placé dans la chambre et comprend un ventilateur (8) qui a un poids de moins de 30 g provoquant un écoulement d'air depuis l'entrée d'air (6) vers la sortie d'air (7),
(c) une préparation sur le transporteur (10) qui est placée dans l'écoulement d'air et comprend un transporteur ayant supporté sur celui-ci une préparation de contrôle des insectes nuisibles comprenant un composé de contrôle des insectes nuisibles qui est modérément volatil à température ordinaire, et
(d) un moyen pour entraîner le ventilateur qui comprend un moteur à courant continu (5) consommant un courant de 25 mA ou moins sans charge appliquée à une tension de 3 V,
(e) une source de courant à batterie sèche pour le moteur (5), et entraînant ledit ventilateur (8) par ledit moteur (5) pour produire un écoulement d'air vers ladite préparation sur le transporteur (10) pour vaporiser et diffuser ledit composé de contrôle des insectes nuisibles et de telle sorte que ledit écoulement d'air est de 0,2 à 6 l/sec au niveau de ladite sortie d'air (7).

11. Procédé selon la revendication 10, comprenant en outre la production d'un moyen pour réguler l'écoulement d'air de telle manière à optimiser le taux de vaporisation de l'ingrédient actif pour la condition d'utilisation ;

12. Procédé selon la revendication 11, dans lequel ledit moyen de régulation est ledit transporteur (10) placé dans l'écoulement d'air ou un régulateur d'écoulement séparé.

13. Procédé selon l'une quelconque des revendications 10 à 12, dans lequel ladite préparation sur le transporteur (10) est un transporteur ayant supporté sur lui la préparation et ledit transporteur a une forme de nid d'abeille, une forme de filet, une forme de fente, une forme de treillis ou une forme comme du papier perforé, ayant une pluralité de trous de ventilation qui sont ouverts à l'écoulement d'air dans la chambre et agencé en un réseau dans le plan perpendiculaire à l'écoulement d'air et est préparé à partir d'un matériau de moulage organique ou inorganique capable de contenir la préparation.

14. Procédé selon l'une quelconque des revendications 10 à 13, dans lequel ledit appareil (61) a un moyen (71) pour alimenter la préparation (72) audit transporteur (70) et ledit moyen a un matériau de gélification contenant ladite préparation.

15. Procédé selon l'une quelconque des revendications 10 à 14, dans lequel le transporteur a une structure de filet (70) ou une structure de nid d'abeille (10a).

16. Procédé selon la revendication 15, dans lequel ladite préparation sur le transporteur (10) est un nid d'abeille ayant supporté sur lui ladite préparation, ledit nid d'abeille ayant un grand nombre de cellules traversantes ayant une taille de cellule de 2 à 5 mm.

17. Procédé selon la revendication 16, dans lequel ledit nid d'abeille a 200 à 2500 cellules traversantes.
